# EUROPÄISCHE PATENTSCHRIFT

(11) **EP 2 665 454 B1**
(45) Veröffentlichungstag und Bekanntmachung des Hinweises auf die Patenterteilung: **10.08.2016**
(21) Anmeldenummer: 12700065.1
(22) Anmeldetag: 04.01.2012
(51) Int. Cl.: A61F 13/15, A61F 13/496

(54) **VERFAHREN ZUM HERSTELLEN EINES HYGIENEARTIKELS**
METHOD FOR PRODUCING A PERSONAL HYGIENE PRODUCT
PROCÉDÉ POUR PRODUIRE UN ARTICLE HYGIÉNIQUE

(30) Priorität: 19.01.2011 DE 102011009550
(43) Veröffentlichungstag der Anmeldung: 27.11.2013
(73) Patentinhaber: Paul Hartmann AG, 89522 Heidenheim (DE)
(72) Erfinder: GASSNER, Oliver, 50674 Köln (DE); OSTERTAG, Wolfgang, 89547 Gerstetten (DE); BEYRLE, Andreas, 89564 Nattheim (DE); ZIEGLER, Danilo, 73179 Ellwangen/Jagst (DE)
(74) Vertreter: DREISS Patentanwälte PartG mbB
(86) Internationale Anmeldenummer: PCT/EP2012/050091
(87) Internationale Veröffentlichungsnummer: WO 2012/098012

(56) Entgegenhaltungen:
- WO-A2-01/66450
- US-A- 5 932 284

## Beschreibung

Die Erfindung betrifft ein Verfahren zum Herstellen absorbierender Hygieneartikel zum einmaligen Gebrauch, insbesondere in Höschenform, in endloser Fertigung in einer schnelllaufenden Herstellungsmaschine, wobei Flachmaterialabschnitte durch Kleberauftrag miteinander gefügt und verklebt werden, wobei der Kleber auf eine in einer Maschinenrichtung geführte Bahn aufgetragen wird.

Wenn im Zuge der Herstellung eines absorbierenden Hygieneartikels Flachmaterialabschnitte miteinander verklebt werden, so stellt sich das Problem, dass eine innige bis zum Rand eines Flachmaterialabschnitts ausgebildete Kleberverbindung nur dann erzielbar ist, wenn dieser Flachmaterialabschnitt zuvor auch bis zum betreffenden Rand beispielsweise mit Sprühkleber beleimt wurde. Da in schnelllaufenden Herstellungsmaschinen mit wenigstens 100 m/min, insbesondere wenigstens 200 m/min und darüber geförderte Bahnen jedoch in der Querrichtung nie ganz exakt geführt werden können, ist die Herstellung einer Kleberbeschichtung exakt bis zum Rand einer Bahn oder eines Flachmaterialabschnitts der Bahn nicht stets möglich, was - wie erwähnt - zu dem Problem führt, dass der betreffende Flachmaterialabschnitt nicht flächenhaft bis zum Rand mit einem anderen Flachmaterialabschnitt verklebt werden kann. Es besteht solchenfalls das Problem oder die Gefahr, dass diese nicht verklebten Ränder sich im Gebrauch des Hygieneartikels abheben und zu einem unangenehmen Tragegefühl und möglicherweise zu Hautirritationen führen. Ein Sprühkleberauftrag, der prozesssicher den gesamten Rand einer zu beschichtenden Bahn oder eines zu beschichtenden Flachmaterialabschnitts der Bahn erfasst, würde auch bei genauer Ausrichtung der Sprühdüse infolge der variablen Querführung der Bahn dazu führen, dass Sprühkleber die Umgebung der Auftragsstelle innerhalb der Herstellungsmaschine unkontrolliert verunreinigt, was mit erheblichen Nachteilen verbunden ist, da Kleberverschmutzungen innerhalb der Maschine nur sehr zeitaufwändig wieder entfernt werden können und außerdem die Prozesssicherheit und die Standzeit eines Produktionszyklus beeinträchtigen.

Beispielsweise stellt sich dieses Problem bei der Herstellung von Hygieneartikeln in Höschenform, bei denen ein Schrittabschnitt mit einem vorderen Bauchabschnitt und einem hinteren Rückenabschnitt, die im Zuge der Herstellung jeweils in Form einer endlosen Bahn gefördert werden, in Überlappung gebracht und dann verklebt wird. Auch in diesem Fall ist es höchst wünschenswert, dass der Schrittabschnitt im Überlappungsbereich an seinen Längsrandabschnitten mit dem Bauchabschnitt bzw. dem Rückenabschnitt randbündig verklebt wird, so dass es nicht zum Aufwerfen von Randabschnitten kommt. Unkontrolliert abstehende Randabschnitte des Schrittabschnitts, wie die des u.a. herkömmlicherweise auf Folien basierenden Backsheets, können zu unkontrollierter Faltenbildung und Hartstellen und nachteiligerweise zu einem unangenehmen Tragegefühl und Hautirritationen führen. Randbündiges Verkleben war aber seither nicht möglich.

Der vorliegenden Erfindung liegt die Aufgabe zugrunde, ein Verfahren der eingangs genannten Art dahingehend zu verbessern, dass in prozesssicherer und wirtschaftlicher Weise eine optimale Kleberverbindung auch in einem Randbereich von miteinander zu fügenden Flachmaterialabschnitten realisierbar ist.

Diese Aufgabe wird bei einem Verfahren der eingangs genannten Art erfindungsgemäß dadurch gelöst, dass zur Bildung eines nur einen Randbereich erfassenden Kleberauftrags bei einem Flachmaterialabschnitt der in Maschinenrichtung geförderten Bahn eine durch ein insbesondere beheiztes Kleberbad laufende Transferrolle verwendet wird, und dass die Transferrolle nur in dem Randbereich gegen den Flachmaterialabschnitt der Bahn abrollt, indem der Flachmaterialabschnitt auf seiner von der Transferrolle abgewandten Seite gegen eine, insbesondere rotierende Führungs- und Transporteinheit der Herstellungsmaschine anliegt und gestützt wird, die topographisch so ausgebildet ist, dass der Flachmaterialabschnitt die Transferrolle nur mit dem Randbereich kontaktiert und im übrigen zumindest geringfügig von der Transferrolle beabstandet ist und dass sich die Transferrolle und die topographisch ausgebildete Führungs- und Transporteinheit quer zur Maschinenrichtung zumindest geringfügig über den Rand des Flachmaterialabschnitts hinauserstrecken.

Es wird also erfindungsgemäß vorgeschlagen, von der an sich bekannten Sprühklebertechnik Abstand zu nehmen und den Kleber unter Verwendung einer Transferrolle auf den oder die Randbereiche der Bahn oder eines Flachmaterialabschnitts der Bahn aufzubringen. Durch die topographische Gestaltung der Führungs- und Transporteinheit für die Bahn oder den Flachmaterialabschnitt der Bahn ist es möglich, den Kleber gezielt nur in einem Randbereich der Bahn oder des Flachmaterialabschnitts der Bahn aufzubringen. Dadurch dass sich die Transferrolle und die Topographie der Führungs- und Transporteinheit quer zur Maschinenrichtung über den Rand des Flachmaterialabschnitts zumindest um das anzunehmende Maß einer Schlingerbewegung hinaus erstreckt, wird erfindungsgemäß erreicht, dass auch bei einer in der Querrichtung variierenden Bahnführung stets ein Kleberauftrag bis zum äußersten Rand der Bahn oder des Flachmaterialabschnitts realisiert wird. Solchenfalls erweist sich eine gewisse schlingernde Bahnführung in Querrichtung für die randständige Beleimung des Flachmaterialabschnitts ganz und gar unkritisch. Da die Transferrolle und die Führungs- und Transporteinheit mit ihrer Topographie einen entsprechenden Bereich in Querrichtung überfangen, wird stets eine Beleimung des Flachmaterialabschnitts bis zum äußersten Rand erreicht.

Die topographische Gestaltung der Fläche der Führungs- und Transporteinheit kann durch eine Oberflächenstrukturierung realisiert werden. Alternativ kann die topographische Gestaltung der Fläche der Führungs- und Transporteinheit durch für den jeweiligen Einsatzzweck abgestimmte separate und auf der Führungs- und Transporteinheit befestigbare Elemente realisiert werden.

In Weiterbildung der Erfindung wird vorgeschlagen, dass die Anlagefläche der Führungs- und Transporteinheit, insbesondere einer rotierenden Führungs- und Transporteinheit, der Herstellungsmaschine dadurch topographisch ausgebildet wird, dass in einem Bereich, in dem die zu beleimenden Randbereiche des Flachmaterialabschnitts zu liegen kommen, Unterlegmittel, insbesondere streifenförmige Unterlegmittel, vorgesehen werden. Diese streifenförmigen Unterlegmittel sind vorzugsweise aus einem zwar druckfesten jedoch gleichwohl beschränkt nachgiebigen Material, wie insbesondere Silikon, gebildet.

Insbesondere vorteilhaft werden Kleber eingesetzt, welche eine große Kohäsion, also große Bindungskräfte innerhalb des Klebers aufweisen, wobei die Kohäsion insbesondere größer ist als die Adhäsion, insbesondere die Adhäsion zu der topographischen Ausbildung der Fläche der den Flachmaterialabschnitt fördernden Führungs- und Transporteinheit. Eine optimierte Abstimmung von Kohäsions- und Adhäsionsverhalten von Kleber und Material der der Transferrolle zugewandten Fläche der Führungs- und Transporteinheit trägt vorteilhaft zu einer Minimierung der Verschmutzungsgefahr der Herstellungsmaschine durch den Kleber bei.

Insbesondere vorteilhaft wird als Kleber ein Hotmeltkleber eingesetzt. Als Kleber können beispielsweise vorzugsweise eingesetzt werden: H20028 oder H 2481 (Bostik Nederland B.V., Zeggeveld 10, 4705 RP Roosendaal, Niederlanden); Technomelt Q2415 oder Technomelt Q5430 (Henkel KGaA, 40191 Düsseldorf, Deutschland).

Die topographische Ausbildung der Fläche der Führungs- und Transporteinheit, insbesondere in Form der streifenförmigen Unterlegmittel mittels Silikon ist insbesondere vorteilhaft beim Einsatz von Hotmeltklebern. Hotmeltkleber weisen gegenüber Silikon eine im Vergleich zur Kohäsion geringere Adhäsion auf, so dass die Gefahr einer kriechenden und zurückbleibenden Verschmutzung verringert wird.

Weiter erweist sich als vorteilhaft, wenn bei der Ausführung des erfindungsgemäßen Verfahrens der Abstand zwischen dem Flachmaterialabschnitt und der Transferrolle außerhalb des zu beleimenden Randbereichs 0,5 -5 mm, insbesondere 0,5 - 2 mm, insbesondere 0,5 - 1,5 mm, insbesondere 0,5 - 1 mm beträgt.

Bei dem randbereichsseitig zu beleimenden Flachmaterialabschnitt kann es sich in vorteilhafter Weise um eine körperabgewandte Backsheetlage des herzustellen Hygieneartikels handeln.

Weiter erweist es sich als vorteilhaft, wenn die Transferrolle zyklisch an den Flachmaterialabschnitt herangeführt und wieder weggeführt wird, um in der Maschinenrichtung einen intermittierenden Kleberauftrag im Randbereich zu erzeugen. Hierfür kann ein an sich beliebiger Mechanismus bei der Transferrolle vorgesehen werden. Beispielsweise könnte die Transferrolle unter Federvorspannung stehend in Richtung auf die Führungs- und Transporteinheit vorgespannt sein und durch zyklisch wirkende Antriebs- oder Stellmittel, insbesondere durch exzentrisch gesteuerte Antriebs- oder Stellmittel entgegen der Federvorspannung zurückbewegt werden.

Weiter kann es sich als vorteilhaft erweisen, wenn die Führungs- und Transporteinheit der Herstellungsmaschine eine einer Querschneidstation nachgeordnete Dreheinheit umfasst, so dass eine Drehung des Flachmaterialabschnitts um eine senkrecht auf der Förderebene des Flachmaterialabschnitts stehende Achse ausgeführt wird. Auf diese Weise kann ein Flachmaterialabschnitt mit einer in der Maschinenrichtung erstreckten Randbeleimung um 90° gedreht werden und im Anschluss daran in der vorherigen Querrichtung weiter gefördert und auf andere Flachmaterialabschnitte oder Bahnen appliziert und gefügt werden.

Wie eingangs bereits erwähnt kann das Verfahren zur Herstellung von Hygieneartikeln in Höschenform angewandt werden, wobei solchenfalls der Hygieneartikel mit einem vorderen Bauchabschnitt und einem hinteren Rückenabschnitt ausgebildet wird, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden werden, und mit einem einen Absorptionskörper aufweisenden Schrittabschnitt, der sich in einer Längsrichtung des Hygieneartikels zwischen Bauchabschnitt und Rückenabschnitt erstreckt und diese überlappt, so dass er im Überlappungsbereich an den Bauchabschnitt und an den Rückenabschnitt mittels Kleber unlösbar angefügt werden kann, wobei der Schrittabschnitt, der Bauchabschnitt und der Rückenabschnitt die zu fügenden Materialabschnitte bilden und wobei der nur im Randbereich vorgesehene Kleberauftrag bei dem Schrittabschnitt gebildet wird.

Hierbei erweist es sich weiter als vorteilhaft, wenn die Schrittabschnitte, nach der randseitigen Beleimung um eine senkrecht auf der Förderebene des Flachmaterialabschnitts stehende Achse um 90° gedreht werden und dann in Überlappung mit einer die Rückenabschnitte und einer die Bauchabschnitte bildenden und in der Maschinenrichtung geförderten Bahn gebracht und mit diesen klebend verbunden werden.

Die Erfindung betrifft ferner einen Hygieneartikel in Höschenform mit den Merkmalen des Anspruchs 10. Dadurch, dass die Kleberverbindung in Querrichtung bis zum äußersten Längsrand des Schrittabschnitts im Überlappungsbereich mit dem Bauchabschnitt und mit dem Rückenabschnitt ausgebildet oder erstreckt ist, bleiben die Längsränder des Schrittabschnitts gegen den Bauchabschnitt und gegen den Rückenabschnitt fixiert. Es kommt also nicht zu aufstehenden Längsrändern, die zu Hautirritationen führen können. Es zeigt sich außerdem, dass hierdurch eine Verbesserung des Verbunds zwischen Schrittabschnitt und Bauchabschnitt bzw. Schrittabschnitt und Rückenabschnitt erreicht ist. Insbesondere beim Anziehen der Windelhose ergeben sich nämlich erhebliche schräg nach oben wirkende Zugkräfte, die durch den Benutzer in die Chassismaterialien der Windelhose eingeleitet werden. Hierdurch wird die Fügeverbindung zwischen Schrittabschnitt und Bauchabschnitt sowie Rückenabschnitt stark beansprucht. Diese Beanspruchung führte seither in verstärktem Maße zu den vorausgehend angesprochenen sich abhebenden Längsrändern des Schrittabschnitts und den hiermit verbundenen Folgen. Auch die in Umfangsrichtung verlaufenden Elastifizierungsmittel, die bei Windelhosen gewissermaßen über den gesamten Bauch- und Rückenabschnitt verteilt angeordnet sind, üben auf die Fügeverbindung von Schrittabschnitt und Bauchabschnitt bzw. Rückenabschnitt eine ständige Belastung aus. Insbesondere im oberen, also in Längsrichtung hinteren und vorderen Längsrandende des Schrittabschnitts gestalten sich diese Randprobleme als besonders problematisch. Durch die erfindungsgemäße Ausbildung der Kleberverbindung dergestalt, dass sich der Kleber im Überlappungsbereich bis zum äußeren Rand des Schrittabschnitts erstreckt, kann eine bis zum äußeren Rand wirksame Kleberverbindung zwischen Schrittabschnitt und Bauchabschnitt und Rückenabschnitt hergestellt werden, welche sich auf die vorstehend beschriebene Beanspruchungssituation positiv und stabilisierend auswirkt.

Gegenstand der Erfindung ist auch eine Vorrichtung zur Durchführung des erfindungsgemäßen Verfahrens mit den Merkmalen des Anspruchs 11. Weiterbildungen dieser Vorrichtung sind Gegenstand der Unteransprüche 12-14.

So erweist es sich als vorteilhaft, wenn eine Anlagefläche der Führungs- und Transporteinheit der Herstellungsmaschine dadurch topographisch ausgebildet ist, dass in einem Bereich, in dem die zu beleimenden Randbereiche des Flachmaterialabschnitts zu liegen kommen, Unterlegmittel, insbesondere streifenförmige Unterlegmittel, vorgesehen sind.

Es erweist sich weiter als vorteilhaft, wenn die Transferrolle zyklisch an den Flachmaterialabschnitt heranführbar und wegführbar ist, um einen in der Maschinenrichtung intermittierenden Kleberauftrag im Randbereich zu erzeugen.

Es erweist sich weiter als vorteilhaft, wenn eine Drehung des Flachmaterialabschnitts um eine senkrecht auf der Förderebene des Flachmaterialabschnitts stehende Achse ausführbar ist.

Weitere Merkmale, Einzelheiten und Vorteile der Erfindung ergeben sich aus den beigefügten Patentansprüchen und aus der zeichnerischen Darstellung und der nachfolgenden Beschreibung der Erfindung. In der Zeichnung zeigt:
Figur 1 eine Draufsicht auf einen Hygieneartikel, der unter Ausführung des erfindungsgemäßen Verfahrens hergestellt wird;
Figur 2 eine schematische Seitenansicht einer schnelllaufenden Herstellungsmaschine in einem hier interessierenden Förderabschnitt, in dem eine erfindungsgemäße Beleimung von Randabschnitten einer Bahn ausgeführt wird;
Figur 3 eine schematische Schnittansicht einer Beleimungsstation, bei der eine erfindungsgemäß randseitige Beleimung der Bahn ausgeführt wird.

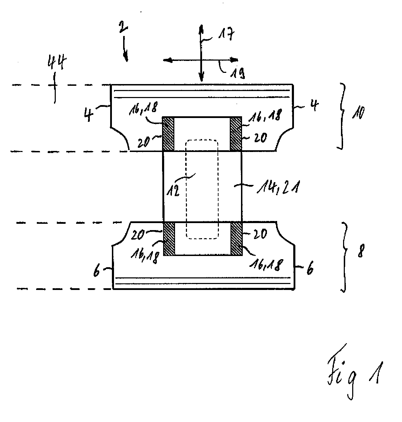

Figur 1 zeigt schematisch eine Draufsicht auf einen Hygieneartikel 2, der nach dem Fügen von Seitennahtbereichen 4,6 eine Höschenform annimmt. Der Hygieneartikel 2 umfasst einen vorderen Bauchabschnitt 8 und einen hinteren Rückenabschnitt 10, die zur Bildung einer in Hüftumfangsrichtung durchgehenden Hüftöffnung an den beidseitigen Seitennahtbereichen 4 und 6 miteinander herstellerseitig unlösbar gefügt werden. Der Hygieneartikel 2 umfasst ferner einen einen Absorptionskörper 12 aufweisenden Schrittabschnitt 14, der über Kleberspuren 16 in Längsrandbereichen 18 mit dem Bauchabschnitt 8 und dem Rückenabschnitt 10 unlösbar gefügt ist. Eine Längsrichtung des Hygieneartikels 2 trägt das Bezugszeichen 17 und eine Querrichtung das Bezugszeichen 19.

Hierbei erweist es sich als erstrebenswert, dass die genannten Kleberspuren 16 in den beidseitigen hinteren und vorderen Randbereichen 18 des Schrittabschnitts 14 in Querrichtung 19 bis zum äußeren Rand 20 des Schrittabschnitts 14 erstreckt sind, so dass beim Fügen des Schrittabschnitts 14 mit dem Bauchabschnitt 8 und dem Rückabschnitt 10 eine innige Klebeverbindung zwischen den beleimten Randbereichen 18 und dem Bauchabschnitt 8 und dem Rückenabschnitt 10 bis zum äußeren Rand 20 realisiert werden kann, damit sich beim Gebrauch des Hygieneartikels der äußere Rand 20 des Schrittabschnitts 14 nicht von dem Bauchabschnitt 8 bzw. Rückenabschnitt 10 erhebt und zu Hautirritationen oder jedenfalls einem unangenehmen Traggefühl führen kann. Dies soll durch Ausführung des erfindungsgemäßen Verfahrens sichergestellt werden.

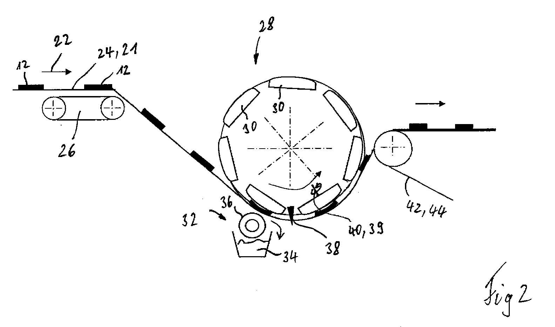

Figur 2 zeigt schematisch eine Seitenansicht einer schnelllaufenden Herstellungsmaschine in einem hier interessierenden Bereich, in dem eine erfindungsgemäße Beleimung der Längsrandbereiche 18 der Schrittabschnitte 14 ausgeführt wird. Zunächst werden die Schrittabschnitte 14 als noch nicht vereinzelte Flachmaterialabschnitte 21 einer in einer Maschinenrichtung 22 endlosen Bahn 24 gefördert. Hierfür ist lediglich schematisch eine Führungsvorrichtung 26, etwa in Form von Unterdruckführungsbändern, schematisch dargestellt. Die Bahn 24 liegt im beispielhaft dargestellten Fall mit ihrer späteren körperabgewandten Seite des Schrittabschnitts 14 gegen die Führungsvorrichtung 26 an. Bei der Bahn 24 handelt es sich beispielsweise um eine das spätere Backsheet des Hygieneartikels 2 im Bereich des Schrittabschnitts 14 bildende Flachmaterialbahn. Ferner dargestellt sind bereits erwähnte Absorptionskörper 12, welche auf die Bahn 24 aufgebracht und bezüglich der Bahn vorzugsweise fixiert worden sind.

Die in der Maschinenrichtung 22 geförderte Bahn 24 wird im weiteren Verlauf des Prozesses mit der späteren körperzugewandten Seite des Hygieneartikels gegen den Umfang einer rotierenden Führungs- und Transporteinheit 28 mit einer Mehrzahl von Unterdruck beaufschlagbaren Schuhen 30 gelegt. Im Bereich dieser rotierenden Führungs- und Transporteinheit 28 ist eine Beleimungsstation 32 mit beispielhaft zwei durch ein insbesondere beheiztes Kleberbad 34 laufenden Transferrollen 36 vorgesehen, mittels derer, auf noch im Einzelnen zu beschreibende Weise, die Kleberspuren 16 in Längsrandbereichen 18 des jeweiligen Flachmaterialabschnitts 21 des Bahn 24 appliziert werden. Der Beleimungsstation nachgeordnet ist eine Querschneidstation 38, bei der senkrecht zur Maschinenrichtung 22 ein Vereinzelungsschnitt bei der Bahn 24 ausgeführt wird. Die auf diese Weise vereinzelten den späteren Schrittabschnitt 14 bildenden Flachmaterialabschnitte 21 werden gleichwohl prozesssicher von den Unterdruck beaufschlagbaren Schuhen 30 gehalten. Ein jeweiliger Schuh 30 ist um eine radiale Achse 40 um 90° drehbar und bildet eine Dreheinheit 39, sodass der an ihm durch Unterdruck fixierte jeweilige Flachmaterialabschnitt 21 ebenfalls um 90° bezüglich der Bahnebene und der Maschinenrichtung 22 gedreht wird. Der Flachmaterialabschnitt 21 bzw. der Schrittabschnitt 14 wird dann nicht mehr in seiner Längsrichtung sondern in seiner Querrichtung (bezogen auf den herzustellenden Hygieneartikel) weiter gefördert. Im Weiteren erfolgt eine Superposition mit einer die Bauchabschnitte 8 und einer die Rückenabschnitte 10 bildenden endlosen Bahn 42 und 44, die auch schematisch in Figur 1 angedeutet ist. Ein jeweiliger Schrittabschnitt 14 wird dabei mit seinen kleberbeschichteten Randbereichen 18 in Überlappung mit den Bahnen 42 und 44 gebracht und daran klebend fixiert.

Im Anschluss an den in Figur 2 rechts dargestellten Fügeprozess wird der Verbund aus endlosen Bahnen 42, 44 und diese überbrückenden Schrittabschnitten 14 in nicht dargestellter, jedoch an sich bekannter Weise um die Längsrichtung auf sich selbst gefaltet und an den Seitennahtbereichen 4, 6 miteinander unlösbar fixiert und schließlich durch einen weiteren Trennvorgang in Querrichtung vereinzelt, was jedoch an sich bekannt ist und daher keiner weiteren Beschreibung bedarf.

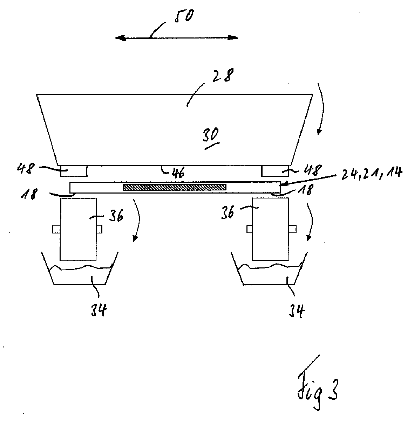

Schließlich zeigt Figur 3 eine schematische Schnittansicht im Bereich der Beleimungsstation 32 senkrecht zur Bahnebene und zur Maschinenrichtung 22. Dargestellt sind beispielhaft zwei voneinander getrennte beheizte Kleberbäder 34 im Bereich der jeweiligen Randbereiche 18 der Bahn 24 sowie je eine Transferrolle 36, welche insbesondere rotierend angetrieben durch das betreffende Kleberbad 34 läuft (zur besseren Darstellung sind die Komponenten in vertikaler Bildrichtung voneinander beabstandet dargestellt). Die Bahn 24 liegt mit ihrer späteren körperzugewandten und den Transferrollen 36 abgewandten Seite gegen einen mit Unterdruck beaufschlagbaren Schuh 30 der rotierenden Führungs- und Transporteinheit 28 an. Eine Anlagefläche 46 der rotierenden Führungs- und Transporteinheit 28 bzw. eines jeweiligen Schuhs 30 der rotierenden Führungs- und Transporteinheit 28 ist topographisch so ausgebildet, dass die Bahn 24 oder ein Flachmaterialabschnitt 21 der Bahn 24 nur in einem Randbereich 18 die Transferrollen 36 kontaktiert. Hierfür ist bei der Anlagefläche 46 ein streifenförmiges Unterlegmittel 48 vorgesehen, durch das die Längsrandbereiche 18 der Bahn 24 gegenüber nicht zu beschichtenden Bereichen der Bahn 24 in Bezug auf den Schuh 30 emporgehoben, also in Richtung auf die Transferrollen 36 exponiert sind. Die Transferrollen 36 als auch die durch die streifenförmigen Unterlegmittel 48 gebildete Topographie der Führungs- und Transporteinheit 28 erstrecken sich in Querrichtung 50 über die seitlichen Ränder 20 der Bahn 24 geringfügig hinaus, sodass auch bei einer mehr oder weniger ausgeprägten schlingernden Querführung der Bahn sichergestellt werden kann, dass unter Verwendung der Transferrollen 36 ein bis zum äußersten Rand 20 gehender Kleberauftrag bei der Bahn 24 realisiert werden kann.

## Patentansprüche

1. Verfahren zum Herstellen absorbierender Hygieneartikel (2) zum einmaligen Gebrauch, insbesondere in Höschenform, in endloser Fertigung in einer schnelllaufenden Herstellungsmaschine, wobei Flachmaterialabschnitte (21) durch Kleberauftrag miteinander gefügt und verklebt werden, wobei der Kleber auf eine in einer Maschinenrichtung geführte Bahn (24) aufgetragen wird, **dadurch gekennzeichnet, dass** zur Bildung eines nur einen Randbereich (18) erfassenden Kleberauftrags bei einem Flachmaterialabschnitt (21) der in Maschinenrichtung (22) geförderten Bahn (24) eine durch ein insbesondere beheiztes Kleberbad (34) laufende Transferrolle (36) verwendet wird, und dass die Transferrolle (36) nur in dem Randbereich (18) gegen den Flachmaterialabschnitt (21) der Bahn (24) abrollt, indem der Flachmaterialabschnitt (21) auf seiner von der Transferrolle (36) abgewandten Seite gegen eine, insbesondere rotierende Führungs- und Transporteinheit (28) der Herstellungsmaschine anliegt und gestützt wird, die topographisch so ausgebildet ist, dass der Flachmaterialabschnitt (21) die Transferrolle (36) nur mit dem Randbereich (18) kontaktiert und im übrigen zumindest geringfügig von der Transferrolle (36) beabstandet ist, und dass sich die Transferrolle (36) und die topographisch ausgebildete Führungs- und Transporteinheit (28) quer zur Maschinenrichtung (22) über einen Rand (20) des Flachmaterialabschnitts (21) hinauserstrecken.

2. Verfahren nach Anspruch 1, **dadurch gekennzeichnet, dass** eine Anlagefläche (46) der Führungs- und Transporteinheit (28) der Herstellungsmaschine dadurch topographisch ausgebildet wird, dass in einem Bereich, in dem die zu beleimenden Randbereiche (18) des Flachmaterialabschnitts (21) zu liegen kommen, Unterlegmittel (48), insbesondere streifenförmige Unterlegmittel (48), vorgesehen werden.

3. Verfahren nach Anspruch 2, **dadurch gekennzeichnet, dass** das streifenförmige Unterlegmittel (48) aus einem beschränkt nachgiebigen Material, wie insbesondere Silikon gebildet ist.

4. Verfahren nach Anspruch 1, 2 oder 3, **dadurch gekennzeichnet, dass** der Abstand zwischen dem Flachmaterialabschnitt (21) und der Transferrolle (36) außerhalb des zu beleimenden Randbereichs (18) 0,5 - 5 mm, insbesondere 0,5 - 2 mm, insbesondere 0,5 - 1,5 mm, insbesondere 0,5 - 1 mm beträgt.

5. Verfahren nach einem oder mehrere der vorstehenden Ansprüche, **dadurch gekennzeichnet, dass** der randbereichsseitig zu beleimende Flachmaterialabschnitt (21) eine körperabgewandte Backsheetlage des Hygieneartikels (2) bildet.

6. Verfahren nach einem oder mehrere der vorstehenden Ansprüche, **dadurch gekennzeichnet, dass** die Transferrolle (36) zyklisch an den Flachmaterialabschnitt (21) herangeführt und weggeführt wird, um in der Maschinenrichtung (22) einen intermittierenden Kleberauftrag im Randbereich (18) zu erzeugen.

7. Verfahren nach einem oder mehrere der vorstehenden Ansprüche, **dadurch gekennzeichnet, dass** die Führungs- und Transporteinheit (28) der Herstellungsmaschine eine einer Querschneidstation (38) nachgeordnete Dreheinheit (39) umfasst, so dass eine Drehung des Flachmaterialabschnitts (21) um eine senkrecht auf der Förderebene des Flachmaterialabschnitts (21) stehende Achse (40) ausgeführt wird.

8. Verfahren nach einem oder mehrere der vorstehenden Ansprüche, **dadurch gekennzeichnet, dass** der Hygieneartikel (2) in Höschenform und mit einem vorderen Bauchabschnitt (8) und einem hinteren Rückenabschnitt (10) ausgebildet wird, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen (4, 6) herstellerseitig miteinander verbunden werden, und mit einem einen Absorptionskörper (12) aufweisenden Schrittabschnitt (14), der sich in einer Längsrichtung (17) des Hygieneartikels (2) zwischen Bauchabschnitt (8) und Rückenabschnitt (10) erstreckt und diese überlappt, so dass er im Überlappungsbereich an den Bauchabschnitt (8) und an den Rückenabschnitt (10) mittels Kleber unlösbar angefügt werden kann, wobei der Schrittabschnitt (14), der Bauchabschnitt (8) und der Rückenabschnitt (10) die zu fügenden Flachmaterialabschnitte (21) bilden und dass der nur im Randbereich (18) vorgesehene Kleberauftrag bei dem Schrittabschnitt (14) gebildet wird.

9. Verfahren nach Anspruch 8, **dadurch gekennzeichnet, dass** die Schrittabschnitte (14) nach der randseitigen Beleimung um eine senkrecht auf der Förderebene des Schrittabschnitts (14) oder Flachmaterialabschnitts (21) stehende Achse (40) um 90° gedreht werden und dann in Überlappung mit einer die Rückenabschnitte (10) und einer die Bauchabschnitte (8) bildenden und in der Maschinenrichtung geförderten Bahn (44, 42) gebracht und mit diesen klebend verbunden werden.

10. Hygieneartikel (2) in Höschenform, mit einem vorderen Bauchabschnitt (8) und einem hinteren Rückenabschnitt (10), die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen (4, 6) herstellerseitig miteinander verbunden sind, und mit einem einen Absorptionskörper (12) aufweisenden Schrittabschnitt (14), der sich in einer Längsrichtung (17) zwischen Bauchabschnitt (6) und Rückenabschnitt (8) erstreckt und diese überlappt und im Überlappungsbereich an den Bauchabschnitt (6) und an den Rückenabschnitt (8) mittels Kleber unlösbar angefügt ist, wobei die Kleberverbindung zwischen Längsrandbereichen (18) des Schrittabschnitts (14) und dem Bauchabschnitt (6) und Rückenabschnitt (8) ausgebildet ist, **dadurch gekennzeichnet, dass** in beidseitigen hinteren und vorderen Längsrandbereichen (18) des Schrittabschnitts (14) in Längsrichtung (17).durch Abrollen von Transferrollen auf den Schrittabschnitt (14) applizierte und in Längsrichtung erstreckte Kleberspuren (16) in Querrichtung (19) bis zum äußeren Längsrand (20) des Schrittabschnitts (14) erstreckt sind, so dass die Kleberverbindung zwischen den beidseitigen Längsrandbereichen (18) des Schrittabschnitts (14) und dem Bauchabschnitt (6) und dem Rückenabschnitt (8) in Querrichtung (19) bis zum äußersten Längsrand (20) des Schrittabschnitts (14) ausgebildet ist und sich keine Längsränder (20) des Schrittabschnitts (14) von dem Bauchabschnitt (6) oder Rückenabschnitt (8) emporheben und zu Hautirritationen führen können.

11. Vorrichtung zur Durchführung eines Verfahrens nach einem oder mehreren der vorstehenden Ansprüche, **gekennzeichnet durch** ein insbesondere beheiztes Kleberbad (34) und eine durch das Kleberbad (34) laufende Transferrolle (36), und **dadurch** dass die Transferrolle (36) nur in dem Randbereich (18) gegen den Flachmaterialabschnitt (21) der Bahn (24) abrollt, und dass eine Führungs- und Transporteinheit (28) für die Bahn (24) vorgesehen ist, gegen die der Flachmaterialabschnitt (21) der Bahn (24) auf seiner von der Transferrolle (36) abgewandten Seite anliegt und gestützt wird, und dass die Führungs- und Transporteinheit (28) topographisch so ausgebildet ist, dass der Flachmaterialabschnitt (21) die Transferrolle (36) nur mit dem Randbereich (18) kontaktiert und im übrigen zumindest geringfügig von der Transferrolle (36) beabstandet ist, und dass die Transferrolle (36) und die topographisch ausgebildete Führungs- und Transporteinheit (28) so ausgebildet und angeordnet sind, dass sie sich quer zur Maschinenrichtung (22) über den Rand (20) des Flachmaterialabschnitts (21) hinauserstreckt.

12. Vorrichtung nach Anspruch 11, **dadurch gekennzeichnet, dass** eine Anlagefläche (46) der Führungs- und Transporteinheit (28) der Herstellungsmaschine dadurch topographisch ausgebildet ist, dass in einem Bereich, in dem die zu beleimenden Randbereiche (18) des Flachmaterialabschnitts (21) zu liegen kommen, Unterlegmittel (48), insbesondere streifenförmige Unterlegmittel (48), vorgesehen sind.

13. Vorrichtung nach Anspruch 11 oder 12, **dadurch gekennzeichnet, dass** die Transferrolle (36) zyklisch an den Flachmaterialabschnitt (21) heranführbar und wegführbar ist, um einen in der Maschinenrichtung (22) intermittierenden Kleberauftrag im Randbereich (18) zu erzeugen.

14. Vorrichtung nach Anspruch 11, 12 oder 13, **dadurch gekennzeichnet, dass** die Führungs- und Transporteinheit (28) der Herstellungsmaschine eine einer Querschneidstation (38) nachgeordnete Dreheinheit (39) umfasst, so dass eine Drehung des Flachmaterialabschnitts (21) um eine senkrecht auf der Förderebene des Flachmaterialabschnitts (21) stehende Achse (40) ausführbar ist.

## Claims

1. A method for producing absorbent personal hygiene products (2) for a single use, in particular in the form of underpants, in endless manufacture in a high-speed production machine, wherein flat-material portions (21) are joined and glued together by applying adhesive, and the adhesive is applied to a length of material (24) guided in a machine direction, **characterized in that** for forming an adhesive application covering only a edge region (18) in a flat-material portion (21) of the length (24) being fed in the machine direction (22), a transfer roller (36) running through an adhesive bath (34), which bath is in particular heated, is used; and that the transfer roller (36) rolls on the flat-material portion (21) of the length (24) only in the edge region (18); that the flat-material portion (21), on its side remote from the transfer roller (36), rests on and is supported by what is in particular a rotating guidance and transfer unit (28) that is embodied topographically such that the flat-material portion (21) contacts the transfer roller (36) only by the edge region (18) and otherwise is at least slightly spaced apart from the transfer roller (36); and that the transfer roller (36) and the topographically embodied guidance and transfer unit (28) extend, transversely to the machine direction (22), past an edge (20) of the flat-material portion (21).

2. The method of claim 1, **characterized in that** a contact surface (46) of the guidance and transfer unit (28) of the production machine is embodied topographically by providing that shim means (48), in particular striplike shim means (48), are provided in a region in which the edge regions (18) of the flat-material portion (21) that are to be glued come to rest.

3. The method of claim 2, **characterized in that** the striplike shim means (48) is formed of a limitedly resilient material, such as silicone in particular.

4. The method of claim 1, 2 or 3, **characterized in that** the spacing between the flat-material portion (21) and the transfer roller (36), outside the edge region (18) to be glued, amounts to from 0.5 to 5 mm, in particular 0.5 to 2 mm, in particular 0.5 to 1.5 mm, in particular 0.5 to 1 mm.

5. The method of one or more of the foregoing claims, **characterized in that** the flat-material portion (21) to be glued on the edge region forms a backing-sheet layer, facing away from the body, of the personal hygiene product (2).

6. The method of one or more of the foregoing claims, **characterized in that** the transfer roller (36) is cyclically brought to and guided away from the flat-material portion (21), in order to generate an intermittent application of glue in the edge region (18) in the machine direction (22).

7. The method of one or more of the foregoing claims, **characterized in that** the guidance and transfer unit (28) of the production machine includes a rotary unit (39), downstream of a transverse cutting station (38), so that a rotation of the flat-material portion (21) about an axis (40) that is vertical to the feeding plane of the flat-material portion (21) is executed.

8. The method of one or more of the foregoing claims, **characterized in that** the personal hygiene product (2) is embodied in the form of underpants and with a front abdominal portion (8) and a rear back portion (10), which are connected together by the manufacturer to form an abdominal and back band that is continuous in the transverse or hip circumference direction, having a closed hip opening, in the hip circumference direction, on side seam regions (4, 6) on both sides, and having a crotch portion (14) which has an absorption body (12) and extends in a longitudinal direction (17) of the personal hygiene product (2) between the abdominal portion (8) and the back portion (10), overlapping them so that in the overlapping region it can be joined nondetachably to the abdominal portion (8) and to the back portion (10) by means of adhesive, and the crotch portion (14), the abdominal portion (8) and the back portion (10) form the flat-material portions (21) that are to be joined; and that the adhesive application provided only in the edge region (18) is formed in the crotch portion (14).

9. The method of claim 8, **characterized in that** after the application of glue to the edges, the crotch portions (14) are rotated by 90° about an axis (40) that is vertical to the feeding plane of the crotch portion (14) or flat-material portion (21) and are then made to overlap a length of material (44, 42), which forms the back portions (10) and one of the abdominal portions (8) is fed in the machine direction, and are connected adhesively to them.

10. A personal hygiene product (2) in the form of underpants, with a front abdominal portion (8) and a rear back portion (10), which are connected together by the manufacturer to form an abdominal and back band that is continuous in the transverse or hip circumference direction and has a closed hip opening, in the hip circumference direction, on side seam regions (4, 6) on both sides, and having a crotch portion (14) which has an absorption body (12) and extends in a longitudinal direction (17) between the abdominal portion (6) and the back portion (8), overlapping them so that in the overlapping region it is joined nondetachably to the abdominal portion (6) and to the back portion (8) by means of adhesive, and the adhesive connection is embodied between longitudinal edge regions (18) of the crotch portion (14) and the abdominal portion (6) and back portion (8), **characterized in that** in back and front longitudinal edge regions (18) on both sides of the crotch portion (14) in the longitudinal direction (17), traces (16) of adhesive applied by rolling from transfer rollers onto the crotch portion (14) and extending in the longitudinal direction are extended in the transverse direction (19) as far as the outer longitudinal edge (20) of the crotch portion (14), so that between the longitudinal edge regions (18) on both sides of the crotch portion (14) and of the abdominal portion (6) and the back portion (8), the adhesive connection is embodied in the transverse direction (19) as far as the outermost longitudinal edge (20) of the crotch portion (14), and so that the longitudinal edges (20) of the crotch portion (14) cannot extend above the abdominal portion (6) or back portion (8) and cause skin irritations.

11. An apparatus for performing a method of one or more of the foregoing claims, **characterized by** an adhesive bath (34), which in particular is heated, and by a transfer roller (36) running through the adhesive bath (34), and **characterized in that** the transfer roller (36) rolls against the flat-material portion (21) of the length (24) only in the edge region (18); and that for the length (24) of material, a guidance and transfer unit (28) is provided, on which the flat-material portion (21) of the length (24) rests and is supported on its side facing away from the transfer roller (36); and that the guidance and transfer unit (28) is embodied topographically such that the flat-material portion (21) contacts the transfer roller (36) only by the edge region (18) and otherwise is at least slightly spaced apart from the transfer roller (36); and that the transfer roller (36) and the topographically embodied guidance and transfer unit (28) are embodied and located such that they extend transversely to the machine direction (22) past the edge (20) of the flat-material portion (21).

12. The apparatus of claim 11, **characterized in that** a contact surface (46) of the guidance and transfer unit (28) of the production machine is embodied topographically by providing that shim means (48), in particular striplike shim means (48), are provided in a region in which the edge regions (18) of the flat-material portion (21) that are to be glued come to rest.

13. The apparatus of claim 11 or 12, **characterized in that** the transfer roller (36) can be cyclically brought to and guided away from the flat-material portion (21), in order to generate an intermittent application of glue in the edge region (18) in the machine direction (22).

14. The apparatus of claim 11, 12 or 13, **characterized in that** the guidance and transfer unit (28) of the production machine includes a rotary unit (39), downstream of a transverse cutting station (38), so that a rotation of the flat-material portion (21) about an axis (40) that is vertical to the feeding plane of the flat-material portion (21) can be executed.

## Revendications

1. Procédé de fabrication en continu d'articles hygiéniques (2) absorbants à usage unique, en particulier sous forme de culottes, dans une machine de fabrication à grande vitesse, dans lequel des portions en matériau plat (21) sont assemblées et collées les unes aux autres par application de colle, la colle étant appliquée sur une bande (24) menée dans une direction de machine, **caractérisé par le fait que**, pour former un enduit de colle qui ne couvre qu'une zone marginale (18), dans une portion en matériau plat (21) de la bande (24) transportée dans la direction de machine (22), on utilise un rouleau de transfert (36) qui passe à travers un bain de colle (34) en particulier chauffé, et que ledit rouleau de transfert (36) ne roule que dans la zone marginale (18) contre la portion en matériau plat (21) de la bande (24) **par le fait que**, sur sa face montrant dans la direction opposée au rouleau de transfert (36), ladite portion en matériau plat (21) s'applique et est appuyée contre une unité de guidage et de transport (28) en particulier rotative de la machine de fabrication, qui est réalisée topographiquement de telle manière que la portion en matériau plat (21) n'entre en contact avec le rouleau de transfert (36) que par la zone marginale (18) et est, du reste, espacée, au moins légèrement, du rouleau de transfert (36), et que ledit rouleau de transfert (36) et ladite unité de guidage et de transport (28) réalisée topographiquement s'étendent transversalement à la direction de machine (22) au-delà d'un bord (20) de la portion en matériau plat (21).

2. Procédé selon la revendication 1, **caractérisé par le fait qu'**une surface d'appui (46) de ladite unité de guidage et de transport (28) de la machine de fabrication est réalisée de manière topographique **par le fait que** des moyens d'appui (48), en particulier des moyens d'appui (48) en forme de bandes, sont prévus dans une zone dans laquelle les zones marginales (18) de la portion en matériau plat (21) qui sont à enduire de colle viennent se situer.

3. Procédé selon la revendication 2, **caractérisé par le fait que** ledit moyen d'appui (48) en forme de bande est réalisé dans un matériau qui est souple de manière limitée, telle que, en particulier, la silicone.

4. Procédé selon la revendication 1, 2 ou 3, **caractérisé par le fait que** la distance séparant la portion en matériau plat (21) et le rouleau de transfert (36) à l'extérieur de la zone marginale (18) à enduire de colle est comprise entre 0,5 et 5 mm, en particulier entre 0,5 et 2 mm, en particulier entre 0,5 et 1,5 mm, en particulier entre 0,5 et 1 mm.

5. Procédé selon l'une ou plusieurs des revendications précédentes, **caractérisé par le fait que** la portion en matériau plat (21) qui est à enduire de colle sur la zone marginale forme une couche de feuille arrière de l'article hygiénique (2), qui montre dans la direction opposée au corps.

6. Procédé selon l'une ou plusieurs des revendications précédentes, **caractérisé par le fait que** le rouleau de transfert (36) est rapproché et éloigné de manière cyclique de la portion en matériau plat (21) afin de produire, dans la direction de machine (22), un enduit de colle intermittent dans la zone marginale (18).

7. Procédé selon l'une ou plusieurs des revendications précédentes, **caractérisé par le fait que** ladite unité de guidage et de transport (28) de la machine de fabrication comprend une unité de rotation (39) qui est disposée en aval d'un poste de coupe transversale (38), de sorte qu'une rotation de la portion en matériau plat (21) autour d'un axe (40) perpendiculaire au plan de transport de la portion en matériau plat (21) est réalisée.

8. Procédé selon l'une ou plusieurs des revendications précédentes, **caractérisé par le fait que** ledit article hygiénique (2) est réalisé sous forme de culotte et avec une portion ventrale avant (8) et une portion dorsale arrière (10) qui, pour la formation d'une bande ventrale et dorsale continue dans le sens transversal ou dans le sens du tour de hanches, avec une ouverture de hanche fermée dans le sens du tour de hanches, sont reliées entre elles par le fabricant au niveau de zones de couture latérale (4, 6) situées de part et d'autre, ainsi qu'avec une portion d'entrejambe (14) présentant un corps absorbant (12), qui s'étend dans une direction longitudinale (17) de l'article hygiénique (2) entre la portion ventrale (8) et la portion dorsale (10) et recouvre celles-ci, de sorte qu'elle peut être assemblée de manière inamovible, au moyen de colle, dans la zone de recouvrement, sur la portion ventrale (8) et sur la portion dorsale (10), la portion d'entrejambe (14), la portion ventrale (8) et la portion dorsale (10) formant les portions en matériau plat (21) à assembler, et que l'enduit de colle qui n'est prévu que dans la zone marginale (18) est formé dans la portion d'entrejambe (14).

9. Procédé selon la revendication 8, **caractérisé par le fait que** les portions d'entrejambe (14), après avoir appliqué la colle sur le bord, sont tournées de 90° autour d'un axe (40) perpendiculaire au plan de transport de la portion d'entrejambe (14) ou de la portion en matériau plat (21) et sont, ensuite, mises en recouvrement avec une bande (44) formant les portions dorsales (10) et avec une bande (42) formant les portions ventrales (8), transportées toutes les deux dans la direction de machine, et sont reliées par collage à celles-ci.

10. Article hygiénique (2) sous forme de culotte, comprenant une portion ventrale avant (8) et une portion dorsale arrière (10) qui, pour la formation d'une bande ventrale et dorsale continue dans le sens transversal ou dans le sens du tour de hanches, avec une ouverture de hanche fermée dans le sens du tour de hanches, sont reliées entre elles par le fabricant au niveau de zones de couture latérale (4, 6) situées de part et d'autre, et comprenant une portion d'entrejambe (14) présentant un corps absorbant (12), qui s'étend dans une direction longitudinale (17) entre la portion ventrale (8) et la portion dorsale (10) et recouvre celles-ci, et qui est assemblée de manière inamovible, au moyen de colle, dans la zone de recouvrement, sur la portion ventrale (8) et sur la portion dorsale (10), la liaison par colle étant réalisée entre les zones marginales longitudinales (18) de la portion d'entrejambe (14) et les portions ventrale (8) et dorsale (10), **caractérisé par le fait que** des traces de colle (16) qui sont appliquées sur la portion d'entrejambe (14) dans des zones marginales longitudinales (18) arrière et avant des deux côtés de la portion d'entrejambe (14), dans la direction longitudinale (17), en faisant rouler des rouleaux de transfert, et qui s'étendent dans la direction longitudinale, s'étendent dans la direction transversale (19) jusqu'au bord longitudinal extérieur (20) de la portion d'entrejambe (14) de sorte que la liaison par colle entre les zones marginales longitudinales (18) des deux côtés de la portion d'entrejambe (14) et les portions ventrale (8) et dorsale (10) est réalisée dans la direction transversale (19) jusqu'au bord longitudinal (20) extrême de la portion d'entrejambe (14) et qu'aucun des bords longitudinaux (20) de la portion d'entrejambe (14) ne peut se soulever de la portion ventrale (8) ou de la portion dorsale (10) et entraîner des irritations de la peau.

11. Dispositif de mise en oeuvre d'un procédé selon l'une ou plusieurs des revendications précédentes, **caractérisé par** un bain de colle (34) en particulier chauffé et par un rouleau de transfert (36) qui passe à travers ledit bain de colle (34) ainsi que par le fait que le rouleau de transfert (36) ne roule que dans la zone marginale (18) contre la portion en matériau plat (21) de la bande (24), et qu'une unité de guidage et de transport (28) pour la bande (24) est prévue contre laquelle la portion en matériau plat (21) de la bande (24) s'applique et est appuyée sur sa face montrant dans la direction opposée au rouleau de transfert (36), et que ladite unité de guidage et de transport (28) est réalisée topographiquement de telle manière que la portion en matériau plat (21) n'entre en contact avec le rouleau de transfert (36) que par la zone marginale (18) et est, du reste, espacée, au moins légèrement, du rouleau de transfert (36), et que ledit rouleau de transfert (36) et ladite unité de guidage et de transport (28) réalisée topographiquement sont réalisés et agencés de telle sorte qu'ils s'étendent transversalement à la direction de machine (22) au-delà du bord (20) de la portion en matériau plat (21).

12. Dispositif selon la revendication 11, **caractérisé par le fait qu'**une surface d'appui (46) de ladite unité de guidage et de transport (28) de la machine de fabrication est réalisée de manière topographique **par le fait que** des moyens d'appui (48), en particulier des moyens d'appui (48) en forme de bandes, sont prévus dans une zone dans laquelle les zones marginales (18) de la portion en matériau plat (21) qui sont à enduire de colle viennent se situer.

13. Dispositif selon la revendication 11 ou 12, **caractérisé par le fait que** le rouleau de transfert (36) peut être rapproché et éloigné de manière cyclique de la portion en matériau plat (21) afin de produire, dans la zone marginale (18), un enduit de colle intermittent dans la direction de machine (22).

14. Dispositif selon la revendication 11, 12 ou 13, **caractérisé par le fait que** ladite unité de guidage et de transport (28) de la machine de fabrication comprend une unité de rotation (39) qui est disposée en aval d'un poste de coupe transversale (38), de sorte qu'une rotation de la portion en matériau plat (21) autour d'un axe (40) perpendiculaire au plan de transport de la portion en matériau plat (21) peut être réalisée.
